Europäisches Patentamt

European Patent Office

Office européen des brevets

(19)

(11) **EP 0 725 625 B1**

(12) **EUROPEAN PATENT SPECIFICATION**

(45) Date of publication and mention
of the grant of the patent:
**22.03.2000 Bulletin 2000/12**

(51) Int. Cl.[7]: **A61K 9/00**, A61K 9/48

(86) International application number:
**PCT/GB94/02373**

(21) Application number: **94931117.9**

(22) Date of filing: **28.10.1994**

(87) International publication number:
**WO 95/11667 (04.05.1995 Gazette 1995/19)**

(54) **GELATIN CAPSULE FILL ABLE TO FOAM**

SCHÄUMENDE GELATINKAPSELFÜLLUNG

SUBSTANCE A POUVOIR MOUSSANT GARNISSANT UNE GELULE DE GELATINE

(84) Designated Contracting States:
**CH DE ES FR GB IT LI NL**

(30) Priority: **29.10.1993 GB 9322314**
**07.06.1994 GB 9411388**

(43) Date of publication of application:
**14.08.1996 Bulletin 1996/33**

(73) Proprietor:
**R.P. SCHERER CORPORATION**
**Troy, Michigan 48007-7060 (US)**

(72) Inventors:
• **SUGDEN, Keith**
  **Beverley, North Humberside HU17 0HN (GB)**

• **HUTCHISON, Keith Graeme**
  **Quenington, Gloucestershire GL7 5BY (GB)**

(74) Representative:
**Hitchcock, Esmond Antony et al**
**Lloyd Wise, Tregear & Co.,**
**Commonwealth House,**
**1-19 New Oxford Street**
**London WC1A 1LW (GB)**

(56) References cited:
**FR-A- 2 304 354        FR-A- 2 512 676**
**FR-A- 2 636 532**

**Description**

This invention relates to pharmaceutical products and to their preparation. In particular, the invention relates to encapsulated products which are capable of generating a foam when contacted with water.

It is known to treat reflux oesphagitis by administration of a preparation which on contact with gastric acid generates a carbonated gelatinous foam or raft which floats on the stomach contents. When reflux occurs it is this raft which precedes the stomach contents into the oesophagus thus protecting the mucosa from further irritation. Known preparations of this type include solid preparation in the form of powders or tablets containing alginic acid, sodium bicarbonate and antacid materials or liquid preparations containing sodium alginate, sodium bicarbonate and calcium carbonate marketed under the name GAVISCON (TM Reckitt & Colman Products Ltd). British Patent No. 1524740 discloses such liquid preparations.

US Patent No. 4172120 discloses a preparation including cholestyramine which is retained in the stomach for a prolonged period of time and is therefore more effective in binding duodenally refluxed bile. This preparation includes alginic acid and/or sodium alginate together with sodium bicarbonate which on being swallowed react with gastric acid to form a carbonated raft which holds the cholestryamine sufficiently loosely that it is able to absorb bile acid in the stomach.

The carbonated alginic acid raft type of product is further exemplified by ALGICON (Rhone-Poulenc Rorer) described in European Patent No. 0179858 B1 as containing magnesium alginate, potassium bicarbonate, magnesium carbonate and as antacid materials aluminium hydroxide/ magnesium carbonate co-dried gel.

GB 2222772 discloses pharmaceutical composition comprising ranitidine, alginic acid and a carbonate or bicarbonate. Various oral dosage forms are disclosed including hard gelatin capsules containing a solid fill of the components.

It has now been found that foam generating compositions may be confined within a gelatin capsule if the ingredients are dispersed within an oil or hydrophilic liquid vehicle.

Therefore according to the present invention there is provided a pharmaceutical product in the form of a soft gelatin capsule with a fill comprising a hydrogel material, a gas generator and an oil-based liquid vehicle in which the gas generator comprises bicarbonate having a particle size not exceeding 100μm which is suspended in said oil-based liquid vehicle, wherein upon contact with acidic aqueous medium the soft gelatin capsule breaks up, disperses or dissolves and the fill reacts to form a foam.

The product of the invention is in the form of soft gelatin capsules which may be chewed or swallowed or simply immersed in an aqueous medium. The ingredients of the fill are adapted to produce a foam upon contact with water, particularly upon contact with acidic, relatively low pH water of the kind present in a human stomach. The gas generator is a bicarbonate e.g. sodium bicarbonate. On contact with acid, sodium bicarbonate reacts to produce carbon dioxide which becomes entrapped in the hydrogel material to form the foam.

The products of the invention have a wide range of uses eg therapeutical treatment, delivery of a food supplement and as a dietary product. The term pharmaceutical product herein is used generically to refer to products for all such uses.

The fill may optionally include one or more active ingredients such as, pharmacologically active compound, food supplements, dietary products or any combination thereof. When a capsule reaches the stomach and ruptures, its contents will be released and a foam formed. Alternatively, a capsule may be bitten in the mouth and subsequently swallowed. The active ingredient, such as a drug, will be incorporated in the foam and gradually leached out in the stomach to produce a local or systematic effect. In a medical application, this process has a number of advantages. Primarily, the release of the drug and its absorption from the foam is gradual, resulting in better absorption of the drug. The absorption is also more consistent, since gastric emptying times are less variable following oral administration of the foam producing product. Less variation in gastric emptying times means that the time taken for the drug
to reach the main absorptive region of the upper small intestine is more consistent. This process also provides improved convenience for patients who require concomitant therapy.

Products according to the present invention are particularly useful in the oral administration for the treatment of digestive problems, such as gastrooesophageal reflux, hiatus hernia and heartburn.

However, the products of the invention are not limited to oral administration and the capsules may be added to acidic aqueous medium to generate a foam which is then topically applied or ingested.

Examples of suitable hydrogels include polyuronic acids, such as alginic acid or its salts; pectins; polyacrylic acids such as Carbomer; modified celluloses such as hydroxypropyl methylcellulose; microbial polysaccharides, such as Xanthan gum; gellan and carageenan. Mixtures may also be used.

A feature of certain hydrogels, particularly polyuronic acids, is that in aqueous solution they undergo intermolecular cross-linking with divalent metal ions. For example, polyuronic acids are block co-polymer molecules consisting of poly-mannuronic and poly-guluronic acid residues. Divalent metal ions such as calcium in solution will bind by hydrogen bonding, between matching chain regions of poly-guluronic acid. In an aqueous medium, it is known that polyuronates, such as alginate, form gels in the presence of dissolved divalent metal ions.

Preferably the hydrogel material is the sodium, potassium, ammonium, magnesium or calcium salt of alginic acid or the propylene glycolesters or mixtures thereof.

It has been found that when the polymeric material is alginic acid or a salt of ester thereof foams or rafts of higher strength are obtained if the composition includes a source of divalent calcium or trivalent aluminium ion which act as cross-linking agents. Suitable sources of calcium ions are those derived from the carbonate, lactate, chloride, gluconate, phosphate, hydrogen phosphate, sulphate, tartrate or citrate salts. Suitable sources of aluminium ions are derived from the carbonate, lactate, glycinate or phosphate salts or from aluminium magnesium carbonate hydroxide, magaldrate, aluminium sodium carbonate hydroxide or aluminium sodium silicate. Generally, the relative quantities by weight of the calcium salt or aluminium compound to the alginic acid or alginate calculated as ions are 4 to 120 $Ca^{2+}$ to 500 alginate or 2 to 80 $Al^{3+}$ to 500 alginate respectively.

Suitable oil-based liquid vehicles for use in the invention include hydrogenated natural oils, synthetic oils such as polymethylsiloxane (dimethicone), neutral oils such as fractionated coconut oil, mineral oils, triacetin, ethyl oleate, and other natural oils such as: Soyabean Oil; Arachis Oil; Corn Oil; Sesame Oil; Olive Oil; Rapeseed Oil; Sunflower Oil and Safflower Oil. A preferred oil is fractionated coconut oil.

Hydrophic based liquid vehicles may be used with the oil-based liquid vehicles. Suitable hydrophilic based liquid vehicles for use in the invention include: Polyethylene Glycols (PEGs), particularly PEG 400 and PEG 600: Glycofurol; Polyglycerols; Propylene Glycol; Ethanol; Glycerol; Transcutol; polysorbate and propylene carbonate.

Surprisingly, it has been found that sodium bicarbonate can be dispersed with a hydrogel in an oil-based vehicle without the oil inhibiting the formation of a foam on contact with water. It is also surprising that interaction between sodium bicarbonate and the aqueous gelatin shell to provoke premature gas formation can be avoided even during long term storage. This is particularly relevant when a soft gelatin capsule is used as at the manufacturing stage the water content of the capsule shell is relatively high; for example 20 to 30% by weight, although the water content falls to about, 5 to 10% by weight during storage. Contact between the sodium bicarbonate and the capsule shell can be largely inhibited by efficient mixing to ensure complete coating of the bicarbonate particle surfaces with the oil-based vehicle. The selection of the particle size of the bicarbonate is also significant. The particle size is relevant to the quality of the foam produced, and particle sizes not exceeding 100μm are used.

The gas generator comprises bicarbonate and may include carbonate. Suitable carbonates and bicarbonates include potassium carbonate or bicarbonate, sodium carbonate or bicarbonate, calcium carbonate, sodium glycine carbonate, magnesium carbonate or aluminium carbonate. The bicarbonate (and carbonate) is present in an amount so as to provide an adequate volume of gas (carbon dioxide) to foam the gel produced when the fill contacts the aqueous acidic medium. Generally, the relative quantities by weight of hydrogel material to the bicarbonate (and carbonate) calculated as ions is 35 to 300 $CO_3^{2-}$ or $HCO_{3-}$ to 500 hydrogel material.

It will be understood that the rigidity and thickness of the carbonated foam formed on contact with the aqueous acidic medium may be varied by altering the ratio of carbonate or bicarbonate to the hydrogel material and upon the type of the hydrogel material.

The stability of the suspension of the bicarbonate/carbonate may be improved by the addition of a thickening agent. Suitable thickening agents include colloidal silicon dioxide, such as Aerosil, hydrogenated vegetable fats, glycerol monostearate or glycerol palmitate, and high molecular weight polyethylene glycols eg PEG 1500 to PEG 300.

Another preferred additional ingredient in the contents of capsules of the invention is a surfactant. Efficient dispersion of the oily suspension formulation on contact with the aqueous medium can be enhanced by the use of suitable surfactant system. Surfactants can improve the volume and strength of the foam produced, by maximising the contact with water and allowing the maximum amount of gas to be entrapped. They also increase the opportunity for divalent metal ions to interact with the hydrogel. Suitable surfactants include:

Reaction products of natural or hydrogenated vegetable oils and ethylene glycol; i.e. polyoxyethylene glycolated natural or hydrogenated vegetable oils; e.g. of the type available under the Trade Names CREMOPHOR and NIKKOL;

Polyeoxyethylene-sorbitan-fatty acid esters; e.g. mono- and tri-lauryl, palmityl, stearyl and oleyl esters; e.g. of the type available under the Trade Name TWEEN;

Polyoxyethylene fatty acid esters; e.g. polyoxyethylene stearic acid esters of the type available under the Trade Name MYRJ;

Polyoxyethylene-polyoxypropylene co-polymers; e.g. of the type available under the Trade Names PLURONIC and EMKALYX;

Polyoxyethylene-polyoxypropylene block co-polymers; e.g. of the type available under the Trade Name POLOXAMER;

Dioctylsuccinate, dioctylsodiumsulfosuccinate, di-(2-ethylhexyl)-succinate or sodium lauryl sulfate;

Phospholipids, in particular lecithins; Propylene glycol mono- and di-fatty acid esters such as propylene glycol dicaprylate; propylene glycol dilaurate, propylene glycol hydroxystearate, propylene glycol isostearate, propylene glycol laurate, propylene glycol ricinoleate, and propylene glycol stearate, most preferably propylene glycol caprylic-capric acid diester as is available under the Trade Name MIGLYOL 840; Bile salts, e.g. alkali metal salts such as sodium taurocholate;

Trans-esterification products of natural vegetable oil triglycerides and polyalkylene polyols (e.g. LABRAFIL);
Mono-, di- and mono/di-glycerides, especially esterification products of caprylic or capric acid with glycerol; e.g. of the type available under the Trade Name IMWITOR;
Sorbitan fatty acid esters e.g. of the type available under the Trade Name SPAN, including sorbitan-monolauryl, -monopalmityl, -monostearyl, -tristearyl, -monooleyl and trioleyl esters;

Monoglycerides, e.g. glycerol monooleate, glycerol monopalmitate and glycerol monostearate, for example of the type available under the Trade Names MYVATEX, MYVAPLEX and MYVEROL, and acetylated, e.g. mono- and di-acetylated monoglycerides, for example those available under the Trade Name MYVACET;
Glycerol triacetate or (1,2,3)-triacetin.

Capsules of the invention can include flavouring and aromatic components, in the fill and/or in the capsule shell material itself. Suitable components include essential oils such as lemon, orange and peppermint oils; fruit flavours; aniseed; liquorice; caramel; honey; cream; various spices and combinations of these and other flavours. Such components are supplied by International Flavours & Fragrances, IFF (GB) Ltd. of Haverhill, Suffolk, CB9 8LG ENGLAND.

Natural or artificial sweeteners can also be used, such as; Aspartame, Saccharin, Acesulphame K, Neohesperidine hydrochloride, Mannitol, Xylitol, and Maltitol;

Taste-masking ingredients such as ion exchange resins, cyclodextrins and adsorbates may also be used.

The gelatin capsules may be simultaneously formed and filled using conventional methods and apparatus such as disclosed, for example, in an article by H. Seager in Pharmaceutical Technology September 1985.

The fill is generally prepared by admixing the hydrogel material and gas generator with the liquid vehicle. The thickener is generally added after the initial admixture.

A high speed mixer or colloidal mill is preferably used to ensure a thorough dispersion is obtained. Heating may be employed when necessary.

The encapsulation machine is suitably an R.P. Scherer encapsulation machine.

The invention will now be illustrated by the following Examples.

Example 1

A basic formulation for the contents of a soft gelatin capsule embodying the present invention is as follows

|  | Quantity per Capsule |
| --- | --- |
| Sodium Alginate | 500mg |
| Calcium Carbonate | 160mg |
| Sodium Bicarbonate | 270mg |
| Fractionated Coconut Oil | 580mg |

On addition of this formulation to an acidic aqueous medium a foam is produced which floats on the surface. The foam is uniformly distributed on the surface and is stable for a substantial period. This formulation can be used as the basis for a therapeutic, pharmaceutical or dietary product, although the proportions may well have to be adjusted to accommodate the additional ingredient or ingredients.

Example 2

| Fill Formulation: | |
|---|---|
| | Quantity per Capsule |
| Alginic Acid | 500mg |
| Sodium Bicarbonate | 540mg |
| Calcium Carbonate | 308mg |
| Fractionated Coconut Oil | 603mg |
| Lecithin, light | 12mg |

Example 3

| Fill Formulation: | |
|---|---|
| | Quantity per Capsule |
| Alginic Acid | 500mg |
| Sodium Bicarbonate | 250mg |
| Calcium Carbonate | 408mg |
| Fractionated Coconut Oil | 743mg |
| Lecithin, light | 15mg |
| Colloidal Silicon Dioxide * | 34mg |
| Sorbitan Fatty Acid Esters ** | 34mg |
| Polysorbate 80 *** | 18mg |

* Aerosil 300
** Span 80
*** Tween 80

Example 4

| Fill Formulation: | |
|---|---|
| | Quantity per Capsule |
| Sodium Alginate | 500mg |
| Calcium Carbonate | 800mg |
| Sodium Bicarbonate | 400mg |
| Fractionated Coconut Oil | 594mg |

(continued)

| Fill Formulation: | |
|---|---|
| | Quantity per Capsule |
| Lecithin | 12mg |
| Carbomer | 3mg |
| Sorbitan Fatty Acid Esters (Span 80) | 34mg |
| Polysorbate 80 | 18mg |

Example 5

| Fill Formulation: | |
|---|---|
| | Quantity per Capsule |
| Sodium Alginate | 500mg |
| Calcium Carbonate | 800mg |
| Sodium Bicarbonate | 400mg |
| Fractionated Coconut Oil | 594mg |
| Lecithin | 12mg |
| Colloidal Silicon Dioxide (Aerosil) | 34mg |
| Sorbitan Fatty Acid Esters (Span 80) | 34mg |
| Polysorbate 80 | 18mg |

Example 6

Capsules in a standard gelatine shell were prepared having the following fill weights:

| Fill Formulation | |
|---|---|
| Sodium Alginate | 500mg |
| Sodium Bicarbonate BP | 100mg |
| Calcium Carbonate | 30mg |
| Fractionated Coconut Oil | 600mg |
| Lecithin | 12mg |
| Colloidal Silicon Dioxide | 34mg |
| Sorbitan Fatty Acid Esters | 34mg |
| Polysorbate 80 | 20mg |
| Flavouring, colouring, sweetener | 80mg |

Example 7

Capsules were prepared as in Example 6 except the amount of calcium carbonate in the fill formulation was increased to 100mg.

Example 8

Capsules were prepared as in Example 6 having the following fill:

|  | Quantity/Capsule |
| --- | --- |
| Sodium Alginate | 500mg |
| Xanthan Gum | 100mg |
| Sodium Bicarbonate | 100mg |
| Calcium Carbonate | 100mg |
| Aerosil | 35mg |
| Flavour, Sweetener | qs |
| Soya Bean Oil | qs ad |
|  | 1500mg |

Example 9

Capsules were prepared as in Example 6 having the following fill:

|  | Quantity/Capsule |
| --- | --- |
| Alginic Acid | 500mg |
| Carrageenan | 100mg |
| Sodium Carbonate | 100mg |
| Calcium Chloride | 100mg |
| Aerosil | 35mg |
| Polysorbate 80 | 20mg |
| Flavour, Sweetener | qs |
| Fractionated Coconut oil | qs ad |
|  | 1500mg |

Example 10

Capsules were prepared as in Example 6 having the following fill:

|  | Quantity/Capsule |
| --- | --- |
| Magnesium Alginate | 500mg |
| Gellan Gum | 50mg |
| Magaldrate | 200mg |

(continued)

|  | Quantity/Capsule |
|---|---|
| Sodium Bicarbonate | 150mg |
| Glyceryl Mono-Stearate | 100mg |
| Polysorbate 80 | 20mg |
| Flavour, Sweetener | qs |
| Fractionated Coconut Oil | qs ad |
|  | 1600mg |

Example 11

Capsules were prepared as in Example 6 having the following fill:

|  | Quantity/Capsule |
|---|---|
| Alginic Acid | 300mg |
| Pectin | 300mg |
| Calcium Carbonate | 150mg |
| Sodium Bicarbonate | 150mg |
| Hydrogenated Vegetable Oil | 150mg |
| Lecithin | 15mg |
| Flavour, Sweetener | qs |
| Arachis Oil | qs ad |
|  | 1550mg |

**Claims**

1. A pharmaceutical product in the form of a soft gelatin capsule with a fill comprising a hydrogel material, a gas generator and an oil-based liquid vehicle in which the gas generator comprises bicarbonate having a particle size not exceeding 100μm which is suspended in said oil-based liquid vehicle, wherein upon contact with acidic aqueous medium the soft gelatin capsule breaks up, disperses or dissolves and the fill reacts to form a foam.

2. A product as claimed in Claim 1 in which the hydrogel material is selected from polyuronic acids; pectins; polyacrylic acids; modified cellulose and microbial polysaccharides.

3. A product as claimed in Claim 1 or Claim 2 in which the hydrogel material is selected from alginic acid, alginates, pectin, xanthan, gellan, carageenan and mixtures thereof.

4. A product as claimed in Claim 3 wherein the hydrogel material is alginic acid or the alginates, are the sodium, potassium, ammonium, magnesium or calcium salts or the propylene glycol esters or mixtures thereof.

5. A product as claimed in Claim 3 which includes a calcium or aluminium cross-linking ion.

6. A product as claimed in Claim 4 wherein the calcium ion is derived from the carbonate, lactate, chloride, gluconate, phosphate, hydrogen phosphate, sulphate, tartrate or citrate salt.

7. A product as claimed in Claim 5 wherein the aluminium ion is derived from the carbonate, lactate, glycinate or phosphate salt or from, aluminium magnesium carbonate hydroxide, magaldrate, aluminium sodium carbonate hydroxide or aluminium sodium silicate.

8. A product as claimed in any preceding Claim in which the bicarbonate is sodium or potassium bicarbonate.

9. A product as claimed in any preceding Claim in which the gas generator comprises a carbonate and bicarbonate.

10. A product as claimed in Claim 9 in which the carbonate is potassium carbonate, sodium carbonate, calcium carbonate, sodium glycine carbonate, magnesium carbonate or aluminium carbonate.

11. A product as claimed in Claim 9 or Claim 10 in which the predominant particle size of the carbonate is no greater than 100μm.

12. A product as claimed in any preceding Claim in which the oil-based liquid vehicle is fractionated coconut oil.

13. A product as claimed in any preceding Claim which additionally comprises a hydrophilic based liquid vehicle.

14. A product as claimed in Claim 13 in which the hydrophilic based liquid vehicle is selected from polyethylene glycol and polypropylene glycol.

15. A product as claimed in any preceding Claim in which the fill includes a surfactant.

16. A product as claimed in any preceding Claim in which the fill includes a thickening agent.

17. A product as claimed in Claim 15 in which the thickening agent is colloidal silicon dioxide.

18. A product as claimed in any preceding Claim in which the fill includes a pharmacologically active compound, a food supplement and/or a dietary product.

19. A method of encapsulating a foam forming mixture comprising a hydrogel material and a gas generator comprising a bicarbonate having a particle size not exceeding 100μm, the method comprising admixing said foam forming mixture with an oil-based liquid vehicle to form a fill formulation in which the bicarbonate is suspended in said oil-based liquid vehicle and encapsulating the fill formulation in a soft gelatin capsule.

20. A method as claimed in Claim 19 in which the components of the fill formulations are as defined in any one of Claims 2 to 18.

**Patentansprüche**

1. Pharmazeutisches Produkt in Form einer Weichgelatinekapsel mit einer Füllung, welche ein Hydrogel-Material, einen Gaserzeuger und ein flüssiges Vehikel auf Öl-Basis umfaßt, wobei der Gaserzeuger Bicarbonat mit einer Partikelgröße von nicht mehr als 100 μm umfaßt, welches im flüssigen Vehikel auf Öl-Basis suspendiert ist, wobei die Weichgelatinekapsel bei Kontakt mit saurem wäßrigen Medium aufbricht, sich dispergiert oder löst und die Füllung unter Bildung eines Schaums reagiert.

2. Produkt wie in Anspruch 1 beansprucht, wobei das Hydrogel-Material ausgewählt wird aus Polyuronsäuren; Pektinen; Polyacrylsäuren; modifizierter Cellulose und mikrobiellen Polysacchariden.

3. Produkt wie in Anspruch 1 oder Anspruch 2 beansprucht, wobei das Hydrogel-Material ausgewählt wird aus Alginsäure, Alginaten, Pektin, Xanthan, Gellan, Karragheen und Mischungen davon.

4. Produkt wie in Anspruch 3 beansprucht, wobei das Hydrogel-Material Alginsäure oder die Alginate ist, die Natrium-, Kalium-, Ammonium-, Magnesium- oder Calciumsalze oder die Propylenglykolester oder Mischungen davon sind.

5. Produkt wie in Anspruch 3 beansprucht, welches ein vernetzendes Calcium- oder Aluminiumion einschließt.

6. Produkt wie in Anspruch 4 beansprucht, wobei das Calciumion erhalten wird aus dem Carbonat-, Lactat-, Chlorid-, Glykonat-, Phosphat-, Hydrogenphosphat-, Sulfat-, Tartrat- oder Citratsalz.

7. Produkt wie in Anspruch 5 beansprucht, wobei das Aluminiumion erhalten wird aus dem Carbonat-, Lactat-, Glycinat- oder Phosphatsalz oder aus Aluminium-Magnesium-Carbonat-Hydroxid, Magaldrat, Aluminium-Natrium-Carbonat-Hydroxid oder Aluminium-Natrium-Silikat.

8. Produkt wie in einem der vorstehenden Ansprüche beansprucht, wobei das Bicarbonat Natrium- oder Kaliumbicarbonat ist.

9. Produkt wie in einem der vorstehenden Ansprüche beansprucht, wobei der Gaserzeuger ein Carbonat und Bicarbonat umfaßt.

10. Produkt wie in Anspruch 9 beansprucht, wobei das Carbonat Kaliumcarbonat, Natriumcarbonat, Calciumcarbonat, Natrium-Glycin-Carbonat, Magnesiumcarbonat oder Aluminiumcarbonat ist.

11. Produkt wie in Anspruch 9 oder Anspruch 10 beansprucht, wobei die vorherrschenden Partikelgröße des Carbonats nicht größer ist als 100 μm.

12. Produkt wie in einem der vorstehenden Ansprüche beansprucht, wobei das flüssige Vehikel auf Öl-Basis fraktioniertes Kokosöl ist.

13. Produkt wie in einem der vorstehenden Ansprüche beansprucht, welches zusätzlich ein flüssiges hydrophiles Vehikel umfaßt.

14. Produkt wie in Anspruch 13 beansprucht, wobei das hydrophile flüssige Vehikel ausgewählt wird aus Polyethylenglykol und Polypropylenglykol.

15. Produkt wie in einem der vorstehenden Ansprüche beansprucht, wobei die Füllung einen oberflächenaktiven Stoff einschließt.

16. Produkt wie in einem vorstehenden Anspruch beansprucht, wobei die Füllung einen Verdicker einschließt.

17. Produkt wie in Anspruch 15 beansprucht, wobei der Verdicker kolloidales Siliziumdioxid ist.

18. Produkt wie in einem vorstehenden Anspruch beansprucht, wobei die Füllung eine pharmakologisch wirksame Verbindung, einen Lebensmittelzusatz und/oder ein Diätprodukt einschließt.

19. Verfahren zur Einkapselung einer schaumbildenden Mischung, umfassend ein Hydrogel-Material und einen Gaserzeuger, welcher ein Bicarbonat mit einer Partikelgröße von nicht mehr als 100 μm, wobei das Verfahren Mischen dieser schaumbildenden Mischung mit einem flüssigen Vehikel auf Öl-Basis zur Bildung einer Füllungsformulierung, in der das Bicarbonat in dem flüssigen Vehikel auf Öl-Basis suspendiert ist, und Einkapseln der Füllungsformulierung in einer Weichgelatinekapsel umfaßt.

20. Verfahren wie in Anspruch 19 beansprucht, wobei die Komponenten der Füllungsformulierung wie in einem der Ansprüche 2 bis 18 definiert sind.

**Revendications**

1. Produit pharmaceutique sous forme d'une capsule en gélatine molle dont la formulation encapsulée ou charge comprend une substance de type hydrogel, un principe effervescent et un véhicule liquide à base d'huile dans lequel le principe effervescent comprend du bicarbonate de granulométrie non supérieure à 100 μm qui est en suspension dans ledit véhicule liquide à base d'huile, où à la suite de contact avec un milieu aqueux acide, la capsule en gélatine molle se disloque, se disperse ou se dissout et la formulation encapsulée réagit en formant une mousse.

2. Produit selon la revendication 1, dans lequel la substance de type hydrogel est choisie parmi les acides polyuroniques; les pectines; les acides polyacryliques; la cellulose modifiée et les polysaccharides d'origine microbienne.

3. Produit selon la revendication 1 ou la revendication 2, dans lequel la substance de type hydrogel est choisie parmi l'acide alginique, les alginates, la pectine, le xanthane, le gellane, le carraghéénin et leur mélanges.

**4.** Produit selon la revendication 3, dans lequel la substance de type hydrogel est l'acide alginique ou les alginates, ou les sels de sodium, de potassium, d'ammonium, de magnésium ou de calcium correspondants ou les esters de propylène-glycol ou encore leurs mélanges.

**5.** Produit selon la revendication 3, qui comprend un ion capable de se lier par réticulation avec un ion aluminium ou calcium.

**6.** Produit selon la revendication 4, dans lequel l'ion calcium dérive du sel de carbonate, de lactate, de chlorure, de gluconate, de phosphate, d'hydrogénophosphate, de sulfate, de tartrate, ou de citrate.

**7.** Produit selon la revendication 5, dans lequel l'ion aluminium dérive du sel de carbonate, de lactate, de glycinate ou de phosphate ou d'hydroxyde de carbonate de magnésium et d'aluminium, de magaldrate, d'hydroxyde de carbonate de sodium et d'aluminium ou de silicate de sodium et d'aluminium.

**8.** Produit selon l'une quelconque des revendications précédentes, dans lequel le bicarbonate est le bicarbonate de sodium ou de potassium.

**9.** Produit selon l'une quelconque des revendications précédentes, dans lequel le principe effervescent comprend un carbonate et un bicarbonate.

**10.** Produit selon la revendication 9, dans lequel le carbonate est le carbonate de potassium, le carbonate de sodium, le carbonate de calcium, le carbonate de glycine sodique, le carbonate de magnésium ou le carbonate d'aluminium.

**11.** Produit selon la revendication 9 ou la revendication 10, dans lequel la majorité des particules de carbonate ont une taille non supérieure à 100 μm.

**12.** Produit selon l'une quelconque des revendications précédentes, dans lequel le véhicule liquide à base d'huile est de l'huile de noix de coco fractionnée.

**13.** Produit selon l'une quelconque des revendications précédentes, qui comprend en outre un véhicule liquide à base de substance hydrophile.

**14.** Produit selon la revendication 13, dans lequel le véhicule liquide à base de substance hydrophile est choisi parmi le polyéthylène-glycol et le polypropylène-glycol.

**15.** Produit selon l'une quelconque des revendications précédentes, dans lequel la formulation encapsulée comprend un tensioactif.

**16.** Produit selon l'une quelconque des revendications précédentes, dans lequel la formulation encapsulée comprend un agent épaississant.

**17.** Produit selon la revendication 15, dans lequel l'agent épaississant est du dioxyde de silicium colloïdal.

**18.** Produit selon l'une quelconque des revendications précédentes, dans lequel la formulation encapsulée comprend un composé pharmacologiquement actif, un complément alimentaire et/ou un produit diététique.

**19.** Procédé d'encapsulation d'un mélange moussant comprenant une substance de type hydrogel et un principe effervescent comprenant un bicarbonate de granulométrie non supérieure à 100 μm, le procédé comprenant le mélange dudit mélange moussant avec un véhicule liquide à base d'huile pour obtenir une formulation à encapsuler dans laquelle le bicarbonate est en suspension dans ledit véhicule liquide à base d'huile et l'encapsulation de la formulation à encapsuler dans une capsule en gélatine molle.

**20.** Procédé selon la revendication 19, dans lequel les composants des formulations à encapsuler sont tels que définis dans l'une quelconque des revendications 2 à 18.